(19) Europäisches Patentamt

European Patent Office

Office européen des brevets

(11) **EP 0 755 717 B1**

(12) **FASCICULE DE BREVET EUROPEEN**

(45) Date de publication et mention de la délivrance du brevet:
**24.05.2000 Bulletin 2000/21**

(51) Int Cl.⁷: **B01J 29/18**, C07C 5/27

(21) Numéro de dépôt: **96401638.0**

(22) Date de dépôt: **23.07.1996**

(54) **Catalyseurs à base de zéolithe mordénite modifié au cérium, et son utilisation en isomérisation d'une coupe C8 aromatique**

Mit Cerium modifizierte Mordenit-Zeolith-Katalysatoren und ihre Verwendung für die Isomerisierung einer aromatischen C8 Fraktion

Mordenite zeolite catalysts modified with cerium and use thereof for the isomerisation of an aromatic C8 fraction

(84) Etats contractants désignés:
**DE FR GB IT NL**

(30) Priorité: **24.07.1995 FR 9509058**

(43) Date de publication de la demande:
**29.01.1997 Bulletin 1997/05**

(73) Titulaire: **INSTITUT FRANCAIS DU PETROLE**
**92502 Rueil-Malmaison (FR)**

(72) Inventeurs:
• **Benazzi, Eric**
**78360 Montesson (FR)**

• **Marcilly, Christian**
**78800 Houilles (FR)**
• **Alario, Fabio**
**92200 Neuilly sur Seine (FR)**

(74) Mandataire: **Andreeff, François**
**INSTITUT FRANCAIS DU PETROLE**
**4, avenue de Bois-Préau**
**92502 Rueil-Malmaison (FR)**

(56) Documents cités:
EP-A- 0 559 021          FR-A- 2 134 425
US-A- 3 917 565          US-A- 3 917 739
US-A- 4 665 272

## Description

La présente invention concerne un catalyseur et un procédé d'isomérisation d'une coupe C8 aromatique. Le catalyseur selon l'invention est de type alumino-silicate comprenant une zéolithe MORDENITE dont on a modifié la sélectivité et/ou les propriétés catalytiques en désactivant sélectivement la surface externe des cristaux, par formation d'une couche d'oxyde de cérium sur cette dernière, et comprenant au moins un métal du groupe VIII de la classification périodique des éléments (Handbook of Chemistry and Physics, 65$^{ieme}$ édition, 1984-85), et une matrice. En général, la modification de la surface externe des cristaux de la zéolithe s'effectue par imprégnation à sec de la mordénite par une solution alcoolique ou aqueuse d'un sel de cérium.

Actuellement, les catalyseurs utilisés industriellement dans les réactions d'isomérisation de coupes C8 aromatiques sont essentiellement à base de zéolithe ZSM5 (USP-4, 482, 773 par exemple).

L'intérêt de la zéolithe ZSM5 réside dans son excellente sélectivité de forme, qui conduit à une grande sélectivité en paraxylène, la sélectivité vis-à-vis des réactions secondaires indésirables de dismutation restant à un niveau très faible. D'autres zéolithes à ouverture de pores plus large 12MR (ouverture à 12 atomes d'oxygène) ont aussi été utilisées, telle que la mordénite. Les catalyseurs à base de mordénite sont notamment décrits dans le brevet USP-4723051. Cependant, ces zéolithes ne possèdent pas de propriétés de sélectivité géométrique particulières. Ceci se traduit, quelque soit son rapport Si/Al, par des sélectivités en paraxylène plus faibles que celles obtenues pour la zéolithe ZSM5 et surtout par des productions de triméthylbenzènes plus importantes. La production de triméthylbenzènes par dismutation est en effet favorisée dans la mordénite dont le système microporeux est plus ouvert que celui de la ZSM5: les ouvertures sont à 12 oxygènes au lieu de 10 pour la ZSM5.

La demanderesse a donc recherché des catalyseurs améliorés à base de mordénite pour réduire les réactions parasites de production de triméthylbenzènes notamment.

Il a été trouvé que des catalyseurs contenant du cérium sont favorables à l'isomérisation des xylènes.

Des catalyseurs contenant de la mordénite échangée avec du cérium (0,08-4%), du platine ou du palladium sont connus pour l'isomérisation des n-paraffines, selon le brevet U.S. 3,917,739. Mais ils n'ont jamais été décrits pour l'isomérisation des xylènes.

Un objet de la présente invention est un procédé d'isomérisation d'une coupe C8 aromatique contenant des xylènes, avec un catalyseur comprenant une matrice, de la mordénite, du cérium et au moins un métal du groupe VIII.

Un autre objet de la présente invention est un catalyseur comprenant une matrice, de la mordénite sur laquelle a été déposé 7-40% pds (par rapport au poids total de catalyseur) de cérium, et au moins un métal du groupe VIII (de préférence un métal noble, et avantageusement le palladium et/ou le platine).

En effet, la demanderesse a, de plus, découvert que, de façon surprenante, il est possible en réalisant des imprégnations (à sec préféré) de mordénites par des solutions organiques, de préférence alcoolique, ou aqueuse d'au moins un composé (de préférence d'un sel) de cérium d'obtenir des catalyseurs à base de mordénite et de métal du groupe VIII actifs et sélectifs pour la réaction d'isomérisation des C$_8$ aromatiques.

Cette procédure de préparation nouvelle confère à la mordénite ainsi traitée, dont la surface externe des cristaux a été inhibée, des propriétés de sélectivité très améliorées. Cela, se traduit par une inhibition surprenante des réactions secondaires indésirables telles que la réaction de dismutation mais aussi par une légère diminution de son activité qui n'est cependant pas préjudiciable au rendement du catalyseur à base de mordénite modifiée selon la présente invention. Cette nouvelle mordénite modifiée conduit par ailleurs à des sélectivités vis à vis des réactions parasites de désalkylation inférieures à celles des catalyseurs basés sur la ZSM5. Les solides ainsi obtenus présentent des performances en isomérisation des C$_8$ aromatiques non seulement meilleures que celles des mordénites de l'art antérieur mais également au moins équivalentes, voire supérieures, aux performances des catalyseurs à base de ZSM5.

La mordénite utilisée dans le catalyseur de la présente invention est fabriquée à partir d'une mordénite soit du type à petits pores soit du type à larges pores, aussi bien synthétisée en milieu hydroxyde que fluorure.

La mordénite du type à petits pores possède une teneur pondérale en sodium par rapport au poids de mordénite sèche généralement comprise entre 4 et 6,5%, un rapport atomique Si/Al global généralement compris entre 4 et 7, un volume de maille élémentaire généralement compris entre 2,76 et 2,80 nm$^3$ (avec 1 nm=10$^{-9}$m) et n'adsorbe habituellement que des molécules de diamètre cinétique inférieur à environ 4,4x10$^{-10}$m.

La mordénite du type à larges pores, synthétisée en milieu OH- ou bien en milieu fluorure, par exemple selon le brevet EP-A-427579 , se distingue de celle du type à petits pores par le fait qu'elle peut adsorber des molécules de diamètre cinétique supérieur à environ 6,6x10$^{-10}$m, donc en particulier des molécules de benzène, que son rapport atomique Si/Al global est généralement compris entre 4,5 et 12.

Avant de sélectiver la mordénite par imprégnation à sec par des solutions aqueuses ou alcooliques de composés (sels) de cérium, il peut être nécessaire d'amener le rapport Si/Al global de la mordénite à des valeurs supérieures à celles obtenues à la synthèse et mentionnées ci-avant, c'est-à-dire supérieure à un rapport Si/Al de 12 dans le cas des mordénites à larges pores et supérieur à 7 dans le cas des mordénites à petits pores. Afin d'obtenir des mordénites désaluminées dans une large gamme de rapport Si/Al, toute technique connue de l'homme du métier pourra être employée comme, par exemple, l'attaque acide directe, la calcination en présence ou non de vapeur d'eau de la forme $NH_4^+$ suivie d'une ou de plusieurs attaques acides, les cycles "calcination(s) - attaque(s) acide(s)". Dans le cas spécifique de la mordénite à petits pores, il faut s'assurer que les traitements retenus ont bien conduit à une ouverture des canaux.

La modification de la sélectivité des mordénites, par imprégnation à sec de solutions de sels de cérium, se fait sur des mordénites de préférence à larges pores dont le rapport Si/Al est compris entre 4,5 et 100 et de préférence entre 4,5 et 45 et de manière encore plus préférée entre 4,5 et 20. Lorsque les mordénites sont du type à petits pores, le rapport Si/Al est 4 à 100, de préférence 4 à 45 et avantageusement entre 4 et 7. Lesdites mordénites sont sous forme $Na^+$, $H^+$, $NH_4^+$ ou sous toute forme mixte combinaisons des trois dernières, et de préférence sur la forme $NH_4^+$. La mise sous forme $NH_4^+$ s'effectue soit sur la mordénite brute de synthèse forme sodique sur laquelle plusieurs échanges ioniques par des solutions concentrées de nitrate d'ammonium (10N) permettent d'obtenir une teneur C en pondérale en sodium par rapport au poids de mordénite sèche généralement inférieure à 2000 ppm, de préférence à 1000 ppm et, de manière encore plus préférée, à 500 ppm, soit sur les mordénites désaluminées, pour lesquelles plusieurs échanges successifs au nitrate d'ammonium permettent d'obtenir la forme $NH_4^+$ de ladite zéolithe.
La modification des propriétés catalytiques des échantillons de mordénite se fait selon un mode opératoire qui comprend 3 étapes.

La première étape consiste à réaliser une calcination de la mordénite sous forme NH4$^+$ à une température comprise en 450°C et 550°C sous air sec ou sous gaz inerte sec.

La deuxième étape consiste alors à réaliser une imprégnation à sec de la mordénite par une solution d'un sel de cérium, parmi lesquels on peut citer à titre d'exemples non limitatifs : $Ce(CH_3COO)_3$, $Ce(CH_3COO)_3.1,5H_2O$, $Ce(NO_3)_3.6H_2O$, $Ce(OH)(NO_3)_3.3H_2O$, (Ce(III)-salycilate de formule chimique $Ce(C_7H_5O_3)_3$), (Ce(III)-2,4-pentanedione de formule chimique, Ce(C5H7O2)3). Le solvant utilisé pour mettre en solution les sels de cérium est celui qui permet d'atteindre les plus grandes solubilités. On peut éventuellement ajouter dans le solvant, un agent complexant tel que par exemple l'acide citrique. Les solvants utilisés sont soit l'eau ou bien des solvants organiques, de préférence les solvants utilisés sont l'alcool éthylique et l'eau. Après, l'imprégnation proprement dite, le solide est séché, par exemple à 100°C dans une étuve ventilée durant une durée comprise entre 4 et 24 heures et de préférence entre 4 et 12 heures.

La troisiéme étape consiste à réaliser une calcination sous air sec de la mordénite, préalablement imprégnée par une solution de cérium et séchée, à une température comprise entre 350°C et 600°C et de préférence entre 450°C et 550°C sous air sec pendant une durée comprise entre 4 heures et 36 heures et de préférence entre 5 et 12 heures.

Il est à noter que le cycle complet comportant les étapes 1, 2 et 3 peut être réalisé autant de fois qu'il est nécessaire. Il est possible également de réaliser plusieurs cycles comportant uniquement les étapes 1 et 2, le dernier cycle étant achevé, une étape terminale 3 de calcination est effectuée.

Selon une première variante de préparation, la zéolithe peut ensuite être soumise au dépôt d'au moins un métal du groupe VIII de préférence choisi dans le groupe formé par le platine et le palladium, puis mise en forme par toute technique connue de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière.
Dans la suite du texte on désignera par le terme support le mélange mordénite + matrice.

D'une façon générale, la mise en forme peut être réalisée avec d'autres matrices que l'alumine, telles que par exemple la magnésie, la silice alumine, les argiles (par exemple kaolin, bentonite) et par d'autres techniques que l'extrusion, telles que le pastillage ou la dragéification.

Selon une seconde variante préférée de préparation, la mordénite chargée en cérium est mélangée à la matrice (telle que précédemment décrite et dans les proportions citées) puis le métal hydrogénant du groupe VIII, de préférence Pt et /ou Pd, peut être déposé sur le support, avant ou après mise en forme, par tout procédé connu de l'homme de l'art et permettant le dépôt du métal sur la mordénite. On peut utiliser la technique d'échange cationique avec compétition où le compétiteur est de préférence le nitrate d'ammonium, le rapport de compétition étant au moins égal à 30 et avantageusement 50 à 200. Dans le cas du platine ou du palladium, on utilise habituellement un complexe tétramine du platine ou un complexe tétramine du palladium : ces derniers se déposeront alors pratiquement en totalité sur la mordénite. Cette technique d'échange cationique peut également être utilisée pour déposer directement le métal sur la poudre de mordénite, avant son mélange éventuel avec une matrice.

Selon une troisième variante également préférée, la mordénite chargée en cérium est mélangée à la matrice. On peut alors déposer le(s) métal(aux) du groupe VIII (le platine et/ou le palladium de préférence) non plus directement sur la mordénite, mais sur la matrice, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. En général après le dépôt de platine et/ou de palladium, le catalyseur est comme précédemment soumis à une calcination puis réduit sous hydrogène comme indiqué ci-dessus.

Enfin, selon une dernière variante, la mordénite chargée en cérium est mélangée à une matrice sur laquelle au moins un métal du groupe VIII a été déposé (par les techniques décrites) puis le mélange est mis en forme.

Le dépôt du métal (ou des métaux) du groupe VIII est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures ; de préférence on opèrera entre 350° et 550°C pendant 2 à 5 heures.

Le catalyseur ainsi obtenu contient entre 2 et 98% (de préférence 3-90%) pds de mordénite par rapport au poids total du catalyseur, entre 0,1 et 40% (de préférence 0,2-25%) pds de cérium par rapport au poids total de catalyseur et entre 0,01 et 3% (de préférence 0,02-2%) pds de métal (métaux) du groupe VIII par rapport au poids de catalyseur.

Un catalyseur préféré contient 7-40% pds de cérium, de préférence 7-25% pds, le cérium ayant été déposé sur la mordénite, et de préférence par imprégnation à sec.

Le catalyseur ne contient pas d'halogène.

Le catalyseur bifonctionnel obtenu par les procédures précédentes est mis en oeuvre dans les réactions d'isomérisation d'une coupe C8 aromatique et en particulier les coupes contenant des xylènes (ortho et/ou méta et/ou para), comprenant par exemple soit uniquement un mélange de xylènes, soit un mélange de xylène(s) et d'éthylbenzène. L'isomérisation des alkylaromatiques, et en particulier des xylènes, revêt une importance commerciale considérable. C'est en général surtout le paraxylène qui est le produit le plus recherché, car il est notammant utilisé comme intermédiaire dans la fabrication des fibres polyesters. On préfère fabriquer le paraxylène en isomérisant le métaxylène, qui lui peut être obtenu par isomérisation de l'orthoxylène L'éthylbenzène qui est difficilement séparable par distillation du mélange des xylènes (les points d'ébullition des différents composés sont très proches), se trouve très souvent dans la charge d'isomérisation des hydrocarbures aromatiques en C8.

Les conditions opératoires du procédé d'isomérisation d'une coupe C8 aromatique effectuée en présence d'au moins un catalyseur selon l'invention sont les suivantes :

- température comprise entre 240 et 600°C, de préférence entre 350 et 510° C,
- pression comprise entre 0,05 et 10 MPa, de préférence entre 0,2 et 3 MPa,
- vitesse spatiale (pph, en masse de charge par unité de charge de catalyseur et par heure), comprise entre 0,5 et 200 h$^{-1}$, de préférence entre 2 et 100 h$^{-1}$,
- rapport molaire hydrogène sur hydrocarbures de la charge (H2/HC) compris entre 0,5 et 12, de préférence entre 2 et 6.

Les exemples qui suivent illustrent l'invention sans toutefois en limiter la portée ; ils sont donnés pour une charge formée de 80% d'orthoxylène et de 20% d'éthylbenzène (% en poids).

### Exemple 1: Catalyseur C1 conforme à l'invention

La matière première utilisée est une mordénite à larges pores, qui possède un rapport Si/Al global de 5,2, un rapport Si/Al de charpente mesuré par infrarouge de 5,3, une teneur pondérale en sodium par rapport au poids de mordénite sèche d'environ 4,2%, un volume de maille élémentaire de 2,794 nm$^3$, un volume poreux à l'azote, mesuré à -196°C et à P/Po=0,19 de 0,163 cm$^3$ de liquide par gramme de mordénite et une surface spécifique, mesurée par la méthode B.E.T de 370 m$^2$/g.

La mordénite est tout d'abord soumise à 3 échanges ioniques dans une solution de NH4NO$_3$ 10N à environ 100°C pendant 4 heures, pour chaque échange. La teneur pondérale en sodium mesurée après ce traitement est ainsi inférieure à 50 ppm.

La mordénite forme NH$_4^+$ obtenue précédemment est ensuite soumise à une calcination sous air sec durant 4 heures à 500°C. Puis, on procède à une imprégnation à sec de 50 g de la mordénite précédemment préparée par 60 ml d'une solution éthanolique contenant 19,4 g du sel Ce(NO$_3$)$_3$.6H$_2$O. Puis, on répète une deuxième fois le cycle d'opérations décrits précédemment. La mordénite modifiée est alors soumise à une calcination finale sous air sec à 550°C durant 4heures.

Le solide obtenu à l'issue des ces traitements est référencé HMCe1. La teneur en cérium est de 25% poids par rapport à la zéolithe.

Ce dernier est ensuite intimement mélangé avec de l'alumine sur laquelle 0,3% poids de platine ont été dispersés. Le produit constitué par le mélange mordénite HMCe1+ alumine contient 40% en poids d'alumine. La teneur pondérale en platine du catalyseur final (contenant HMCe1) est donc d'environ 0,12%.

Le catalyseur ainsi fabriqué est ensuite mis en forme par pastillage, calciné sous air à 550°C durant 2 heures et réduit sous hydrogène à 500°C pendant 3 heures.

Le catalyseur C1, ainsi obtenu, est alors testé en isomérisation du mélange orthoxylène (80% poids) et éthylbenzène (20% poids), sous une pression de 1,2 MPa, avec une vitesse spatiale (pph) de 10 (heure)-1 et un rapport molaire hydrogène sur hydrocarbures (H2/HC) de 4 environ.

Les performances du catalyseur C1 (et des catalyseurs préparés dans les exemples suivants ), reportées dans le tableau I, sont définies par :

$$\text{Approche à l'équilibre de l'o-xylène (AEQ-ox) (\%)} =$$

$$\frac{\text{\% de o-xylène/xylènes dans la charge-\% de o-xylène/xylènes dans l'effluent}}{\text{\% de o-xylène/xylènes dans la charge-\% de o-xylène/xylènes à l'équilibre}} \times 100$$

$$\text{Rendement en C8 aromatique (\%)} =$$

$$\frac{\text{masse de C8 aromatiques et de naphtènes dans la recette}}{\text{masse totale de C8 aromatiques dans la charge}} \times 100$$

$$\text{Sélectivité en dismutation (\%)} =$$

$$\frac{\text{masse de triméthylbenzène + masse de benzène}}{\text{masse des produits}} \times 100$$

## Exemple 2 : Catalyseur C2 conforme à l'invention

La matière première utilisée est la même mordénite, forme NH4+, que celle utilisée dans l'exemple 1. Elle possède donc un rapport Si/Al=5,2 et une teneur pondérale en sodium inférieure à 50 ppm. Puis, dans cet exemple, on fait subir tout d'abord une imprégnation à sec à 50 g de la mordénite précédemment préparée par 60 ml d'une solution aqueuse contenant 38,8 g du sel $Ce(NO_3)_3.6H_2O$. La mordénite modifiée est alors soumise à une calcination finale sous air sec à 550°C durant 4 heures.

Le solide obtenu à l'issue des ces traitements est référencé HMCe2. La teneur en cérium est de 25% poids par rapport à la zéolithe.

Les étapes de mélange de la mordénite et de l'alumine, de dispersion du platine, de mise en forme, de réduction du catalyseur et les catalyseur et les conditions de test d'isomérisation sont identiques à celles décrites dans l'exemple 1.

Les performances du catalyseurs C2, ainsi obtenu ,(dont la teneur en platine est d'environ 0,12%) sont reportées dans le tableau 1.

## Exemple 3 : Catalyseur C3 conforme à l'invention

La matière première utilisée est la même mordénite, forme NH4+, que celle utilisée dans l'exemple 1. Elle possède donc un rapport Si/Al=5,2 et une teneur pondérale en sodium inférieure à 50 ppm. Puis, dans cet exemple, la mordénite subit tout d'abord une calcination sous air sec durant 4 heures à 500°C. Puis, on procède à une imprégnation à sec de 50g de la mordénite précédemment préparée par 60 ml d'une solution éthanolique contenant 22g du sel Ce(C5H7O2)3.3H2O (Ce(III) 2,4-pentanedione). Puis, on répète une deuxième fois le cycle d'opérations décrits précédemment. La mordénite modifiée est alors soumise à une calcination finale sous air sec à 550°C durant 4heures.

Le solide obtenu à l'issue des ces traitements est référencé HMCe3. La teneur en cérium est de 25% poids par rapport à la zéolithe.

Les étapes de mélange de la mordénite et de l'alumine, de dispersion du platine, de mise en forme, de réduction du catalyseur et les catalyseur et les conditions de test d'isomérisation sont identiques à celles décrites dans l'exemple 1.

Les performances du catalyseurs C3 ainsi obtenu (dont la teneur en platine est d'environ 0,12%) sont reportées dans le tableau 1.

## Exemple 4 : Catalyseur C4 non conforme à l'invention

Le catalyseur C4 contient la mordénite, forme H, de rapport Si/Al global de 5,2 utilisée lors de la préparation des catalyseurs C1, C2 et C3. Mais, aucune modification de la mordénite selon l'invention n'est réalisée dans cet exemple.

Les étapes de mélange de la mordénite et de l'alumine, de dispersion du platine, de mise en forme, de réduction du catalyseur et les catalyseurs et les conditions de test d'isomérisation sont identiques à celles décrites dans l'exemple 1.

Les performances du catalyseur C4 ainsi obtenu (dont la teneur en platine est d'environ 0.12%) sont reportées dans le tableau 1 dans lequel il est comparé aux catalyseurs C1, C2 et C3 conformes à l'invention.

TABLEAU 1 :

| Effet des traitements sur les sélectivités à iso-approche à l'équilibre | | | | |
|---|---|---|---|---|
| Catalyseurs | C1 conforme à l'invention | C2 conforme à l'invention | C3 conforme à l'invention | C4 non conforme à l'invention |
| Exemple | 1 | 2 | 2 | 4 |
| % AEQ o-xylène | 94,3 | 94,6 | 94,4 | 94,7 |
| % Rdt $C_8$ aromatiques + naphtènes | 93,9 | 94,2 | 94,1 | 92,2 |
| % Dismutation | 2,7 | 2,4 | 2,6 | 4,4 |

Le tableau I décrit les performances des catalyseurs C1, C2 et C3 comportant de la mordénite sélectivée selon l'invention. L'effet, des modifications par imprégnation à sec de sels de cérium sur les sélectivités est particulièrement mis en évidence.

En effet, les catalyseurs C1, C2, C3 conformes à l'invention sont plus performants que le catalyseur C4 de l'art antérieur. En effet, à iso approche à l'équilibre de l'o-xylène, le rendement en isomérisarion des C8 aromatiques + naphtènes obtenus sur les catalyseurs C1, C2 et C3 est supérieur à celui du catalyseur C4 non conforme à l'invention. D'autre part, dans le cas des mordénites sélectivées selon l'invention (catalyseurs C1, C2 et C3) les réactions secondaires de dismutation des xylènes et de l'éthylbenzène conduisant, entre autres, à la formation de triméthylbenzènes sont fortement inhibées par rapport à ce que l'on obtient en présence de mordénite non sélectivée (catalyseur C4).

## Exemple 5 : Catalyseur C5 conforme à l'invention

La matière première utilisée est une mordénite, qui possède un rapport Si/Al global de 10,5, un rapport Si/Al de charpente mesuré par infrarouge de 11,2, une teneur pondérale en sodium par rapport au poids de mordénite sèche d'environ 3,8%, un volume de maille élémentaire de 2,755 nm$^3$, un volume poreux à l'azote, mesuré à -196°C et à P/Po=0,19 de 0,21 cm$^3$ de liquide par gramme de mordénite et une surface spécifique, mesurée par la méthode B.E.T de 480 m$^2$/g.

La mordénite est tout d'abord soumise à 3 échanges ioniques dans une solution de $NH_4NO_3$ 10N à environ 100°C pendant 4 heures, pour chaque échange. La teneur pondérale en sodium est ainsi inférieure à 50 ppm.

La mordénite forme $NH_4^+$ obtenue précédemment est ensuite soumise à une calcination sous air sec durant 4 heures à 500°C. Puis, on procède à une imprégantion à sec de 50 g de la mordénite précédemment préparée par 60 ml d'une solution éthanolique contenant 15,5 g du sel $Ce(NO_3)_3.6H_2O$. Puis, on répète une deuxième fois le cycle d'opérations décrits précédemment. La mordénite modifiée est alors soumise à une calcination finale sous air sec à 550°C durant 4heures.

Le solide obtenu à l'issue des ces traitements est référencé HMCe5. La teneur en cérium est de 20% poids par rapport à la zéolithe.

Ce dernier est ensuite intimement mélangé avec de l'alumine sur laquelle 0,3% poids de platine ont été dispersés. Le produit constitué par le mélange mordénite HMCe5 + alumine contient 40% en poids d'alumine. La teneur pondérale en platine du catalyseur final (contenant HMCe5) est d'environ 0,12%.

Le catalyseur ainsi fabriqué est ensuite mis en forme par pastillage, calciné sous air à 550°C durant 2 heures et réduit sous hydrogène à 500°C pendant 3 heures.

Le catalyseur C5, ainsi obtenu, est alors testé en isomérisation du mélange orthoxylène (80% poids) et éthylbenzène (20% poids), à une température de 410°C, sous une pression de 1,2 MPa, avec une vitesse spatiale (pph) de 10 (heure)-1 et un rapport molaire hydrogène sur hydrocarbures (H2/HC) de 4 environ.

Les performances du catalyseur C5 (et des catalyseurs préparés dans les exemples suivants), reportées dans le tableau II.

## Exemple 6 : Catalyseur C6 non conforme à l'invention

Le catalyseur C6 contient la mordénite, forme H, de rapport Si/AI global de 10,5 utilisée lors de la préparation du catalyseur C5. Cependant, dans cet exemple, aucun traitement de modification selon l'invention n'est réalisé. Les étapes de mélange de la mordénite et de l'alumine, de dispersion du platine, de mise en forme, de réduction du catalyseur et les catalyseur et les conditions de test d'isomérisation sont identiques à celles décrites dans l'exemple 5.

Les performances du catalyseur C6 ainsi obtenu (dont la teneur en platine est d'environ 0,12%) sont reportées dans le tableau II.

TABLEAU II :

| Effet des traitements à iso-approche à l'équilibre | | |
|---|---|---|
| Catalyseurs | C5 conforme à l'invention | C6 non conforme à l'invention |
| Exemple | 5 | 6 |
| % AEQ o-xylène | 95,2 | 95,5 |
| % Rdt $C_8$ aromatiques + naphtènes | 83,8 | 80,6 |
| % Dismutation | 6,5 | 10,2 |

Le tableau II décrit les performances des catalyseurs C5 conforme à l'invention et C6 non modifié selon l'invention. L'effet, des modifications par imprégnation à sec de sels de cérium sur les sélectivités est particulièrement mis en évidence.

En effet, le catalyseur C5 conforme à l'invention est plus performant que le catalyseur C6 de l'art antérieur. En effet, à iso approche à l'équilibre de l'o-xylène, le rendement en isomérisation des C8 aromatiques + naphtènes obtenus sur les catalyseurs C5 est supérieur à celui du catalyseur C6 non conforme à l'invention. D'autre part, dans le cas du catalyseur comportant la mordénite sélectivée selon l'invention (catalyseurs C5) les réactions secondaires de dismutation des xylènes et de l'éthylbènzene conduisant, entre autres, à la formation de triméthylbenzènes sont fortement inhibées par rapport à ce que l'on obtient en présence de mordénite non sélectivée (catalyseurs C6).

## Revendications

1. Catalyseur comprenant entre 2 et 98% pds de mordénite sur laquelle a été déposé de 7 à 40% poids de cérium, et le catalyseur contenant également de 0,01 à 3% poids d'au moins un métal du groupe VIII, les % étant exprimés par rapport au poids total de catalyseur.

2. Catalyseur selon l'une des revendications précédentes, obtenu par imprégnation à sec de la mordénite par une solution organique ou aqueuse d'au moins un composé du cérium.

3. Catalyseur selon l'une des revendications précédentes, obtenu par imprégnation à sec de la mordénite par une solution alcoolique d'au moins un composé du cérium.

4. Catalyseur selon l'une des revendications précédentes, dans lequel la mordénite est du type à larges pores avec un rapport atomique Si/AI global compris entre 4,5 et 100.

5. Catalyseur selon l'une des revendications précédentes, dans lequel la teneur en modernite est comprise entre 3-90% pds par rapport au poids total de catalyseur.

6. Catalyseur selon l'une des revendications précédentes, dans lequel la teneur en cérium est comprise entre 7 et 25% poids par rapport au poids total de catalyseur.

7. Catalyseur selon l'une des revendications précédentes dans lequel la matrice est choisie dans le groupe formé par l'alumine, la magnésie, la silice-alumine, les argiles.

8. Catalyseur selon l'une des revendications précédentes, obtenu par imprégnation de la mordénite par une solution d'au moins un composé de cérium, dépôt d'au moins un métal du groupe VIII, mélange avec la matrice puis mise en forme.

9. Catalyseur selon l'une des revendications 1 à 7, obtenu par imprégnation de la mordénite par une solution d'au moins un composé de cérium, mélange avec la matrice, dépôt d'au moins un métal du groupe VIII sur la mordénite avant ou après mise en forme.

10. Catalyseur selon l'une des revendication 1 à 7 obtenu par imprégnation de la mordénite par une solution d'au moins un composé de cérium, mélange avec la matrice, dépôt d'au moins un métal du groupe VIII sur la matrice avant ou après mise en forme.

11. Catalyseur selon l'une des revendications 1 à 7, obtenu par imprégnation de la mordénite par une solution d'au moins un composé de cérium, mélange avec la matrice sur laquelle au moins un métal du groupe VIII a été déposé, mise en forme.

12. Catalyseur selon l'une des revendications précédentes, dans lequel le métal du groupe VIIII est choisi dans le groupe constitué par le platine et la palladium.

13. Procédé d'isomérisation d'une coupe C8 aromatique contenant des xylènes, à une température de 240-600° C, une pression de 0,05 - 10 MPa, avec une vitesse spatiale de 0,5-200 $h^{-1}$ et un rapport molaire hydrogène/hydrocarbures de 0,5-12, et en présence d'un catalyseur contenant une matrice, une mordénite, du cérium et au moins un métal du groupe VIII.

14. Procédé selon la revendication 13, opérant à une température de 350-510° C, une pression de 0,2 à 3 MPa, avec une vitesse spaciale de 2-100 $^{h-1}$ et un rapport hydrogène/ hydrocarbures de 2 à 6.

15. Procédé selon l'une des revendications 13 ou 14, dans lequel le catalyseur contient 2-98% pds de mordénite, de 0,1-40% pds de cérium, de 0,01-3% pds d'au moins un métal du groupe VIII, les % étant exprimés par rapport au poids total de catalyseur.

16. Procédé selon la revendication 15, dans lequel le catalyseur contient 7-40% pds de cérium.

17. Procédé selon l'une des revendications 13 à 16, dans lequel le cérium est déposé sur la mordénite.

18. Procédé selon l'une des revendications 13 à 17, dans lequel la mordénite est du type à larges pores avec un rapport atomique Si/Al compris entre 4,5 et 100.

19. Procédé selon l'une des revendications 13 à 18, dans lequel la matrice est choisie dans le groupe constitué par l'alumine, la magnésie, la silice-alumine, les argiles.

20. Procédé selon l'une des revendications 13 à 19, dans lequel le métal noble est choisi dans le groupe constitué par le palladium et le platine.

21. Procédé selon l'une des revendications 13 à 20, dans lequel le cérium est déposé sur la mordénite par imprégnation à sec de la mordénite par une solution organique, aqueuse ou alcoolique d'au moins un composé du cérium.

**Patentansprüche**

1. Katalysator, zwischen 2 und 98 Gew.-% Mordenit umfassend, auf dem zwischen 7 und 40 Gew.-% Cer abgeschieden wurden und der Katalysator auch 0,01 bis 3 Gew.-% wenigstens eines Metalls der Gruppe VIII enthält, wobei die Prozente ausgedrückt sind bezogen auf das Gesamtgewicht des Katalysators.

2. Katalysator nach einem der vorhergehenden Ansprüche, erhalten durch Trockenimprägnierung des Mordenits durch eine organische oder wässrige Lösung wenigstens einer Cerverbindung.

3. Katalysator nach einem der vorhergehenden Ansprüche, erhalten durch Trockenimprägnierung des Mordenits durch eine alkoholische Lösung wenigstens einer Cerverbindung.

4. Katalysator nach einem der vorhegenden Ansprüche, bei dem der Mordenit vom Typ mit großen Poren mit einem Atomgesamtverhältnis Si/Al zwischen 4,5 und 100 ist.

5. Katalysator nach einem der vorhergehenden Ansprüche, bei dem der Gehalt an Mordenit zwischen 3 - 90 Gew.-% , bezogen auf das Gesamtgewicht des Katalysators, liegt.

6. Katalysator nach einem der vorhergehenden Ansprüche, bei dem der Gehalt an Cer zwischen 7 und 25 Gew.-% , bezogen auf das Katalysatorgesamtgewicht, liegt.

7. Katalysator nach einem der vorhergehenden Ansprüche, bei dem die Matrix gewählt ist aus der Gruppe, die durch Aluminiumoxid, Magnesiumoxid, Siliziumoxid-Aluminiumoxid und die Tone gebildet ist.

8. Katalysator nach einem der vorhergehenden Ansprüche, erhalten durch Imprägnieren des Mordenits durch eine Lösung wenigstens einer Cerverbindung, Abscheiden wenigstens eines Metalls der Gruppe VIII, Mischen mit der Matrix und dann Formgebung.

9. Katalysator nach einem der Ansprüche 1 bis 7, erhalten durch Imprägnierung des Mordenits durch eine Lösung wenigstens einer Cerverbindung, Mischen mit der Matrix, Abscheiden wenigstens eines Metalls der Gruppe VIII auf dem Mordenit vor oder nach der Gestaltgebung.

10. Katalysator nach einem der Ansprüche 1 bis 7, erhalten durch Imprägnieren des Mordenits durch eine Lösung wenigstens einer Cerverbindung, Mischen mit der Matrix, Abscheiden wenigstens eines Metalls der Gruppe VIII auf der Matrix vor oder nach der Formgebung.

11. Katalysator nach einem der Ansprüche 1 bis 7, erhalten durch Imprägnieren des Mordenits durch eine Lösung wenigstens einer Cerverbindung, Mischen mit der Matrix, auf der wenigstens ein Metall der Gruppe VIII abgeschieden wurde und dann Gestaltgebung.

12. Katalysator nach einem der vorhergehenden Ansprüche, bei dem das Metall der Gruppe VIII gewählt ist aus der durch Platin und Palladium gebildeten Gruppe.

13. Verfahren zur Isomerierung eines aromatischen C8-Schnitts, der Xylene enthält, bei einer Temperatur von 240-600°C, einem Druck von 0,05-10 MPa und einer Raumgeschwindigkeit von 0,5-200 $h^{-1}$ und einem Molverhältnis von Wasserstoff/Kohlenwasserstoffen von 0,5-12 und in Anwesenheit eines Katalysators, der eine Matrix, einen Mordenit, Cer und wenigstens ein Metall der Gruppe VIII enthält.

14. Verfahren nach Anspruch 13, betreibbar bei einer Temperatur von 350-510°C, einem Druck von 0,2 bis 3 MPa, bei einer Raumgeschwindigkeit von 2-100 $h^{-1}$ und einem Verhältnis von Wasserstoff/Kohlenwasserstoffen von 2 bis 6.

15. Verfahren nach einem der Ansprüche 13 oder 14, bei dem der Katalysator 2 - 98 Gew.-% Mordenit, 0,1 bis 40 Gew.-% Cer, 0,01 - 3 Gew.-% wenigstens eines Metalls der Gruppe VIII enthält, wobei die Prozente ausgedrückt sind bezogen auf das Gesamtgewicht des Katalysators.

16. Verfahren nach Anspruch 15, bei dem der Katalysator 7 - 40 Gew.-% Cer enthält.

17. Verfahren nach einem der Ansprüche 13 bis 16, bei dem das Cer auf den Mordenit abgeschieden wird.

18. Verfahren nach einem der Ansprüche 13 bis 17, bei dem der Mordenit vom Typ mit großen Poren bei einem Atomverhältnis von Si/Al zwischen 4,5 und 100 ist.

19. Verfahren nach einem der Ansprüche 13 bis 18, bei dem die Matrix gewählt ist aus der durch Aluminiumoxid, Magnesiumoxid, Siliziumoxid-Aluminiumoxid, oder die Tone gebildeten Gruppe.

20. Verfahren nach einem der Ansprüche 13 bis 19, bei dem das Edelmetall gewählt ist aus der durch Palladium und Platin gebildeten Gruppe.

21. Verfahren nach einem der Ansprüche 13 bis 20, bei dem das Cer auf den Mordenit durch Trockenimprägnierung des Mordenits durch eine organische, wässrige oder alkoholische Lösung wenigstens einer Cerverbindung abgeschieden wird.

**Claims**

1. A catalyst comprising 2% to 98% by weight of mordenite on which 7% to 40% by weight of cerium has been deposited, the catalyst also containing 0.01% to 3% by weight of at least one metal from group VIII, the percentages being with respect to the total catalyst weight.

2. A catalyst according to claim 1, obtained by dry impregnation of mordenite by an organic or aqueous solution of at least one cerium compound.

3. A catalyst according to claim 1 or claim 2, obtained by dry impregnation of the mordenite by an alcoholic solution of at least one cerium compound.

4. A catalyst according to any one of the preceding claims, in which the mordenite is a large pore mordenite with a global Si/Al ratio in the range 4.5 to 100.

5. A catalyst according to any one of the preceding claims, in which the mordenite content is in the range 3% to 90% by weight with respect to the total catalyst weight.

6. A catalyst according to any one of the preceding claims, in which the cerium content is in the range 7% to 25% by weight with respect to the total catalyst weight.

7. A catalyst according to any one of the preceding claims, in which the matrix is selected from the group formed by alumina, magnesia, silica-alumina and clays.

8. A catalyst according to any one of the preceding claims, obtained by impregnating mordenite with a solution of at least one cerium compound, depositing at least one metal from group VIII, mixing with the matrix then forming.

9. A catalyst according to any one of claims 1 to 7, obtained by impregnating mordenite with a solution of at least one cerium compound, mixing with the matrix, and depositing at least one metal from group VIII on the mordenite before or after forming.

10. A catalyst according to any one of claims 1 to 7, obtained by impregnating mordenite with a solution of at least one cerium compound, mixing with the matrix, and depositing at least one metal from group VIII on the matrix before or after forming.

11. A catalyst according to any one of claims 1 to 7, obtained by impregnating mordenite with a solution of at least one cerium compound, mixing with the matrix on which at least one metal from group VIII has been deposited, then forming.

12. A catalyst according to any one of the preceding claims, in which the metal from group VIII is selected from the group constituted by platinum and palladium.

13. A process for the isomerisation of an aromatic C8 cut containing xylenes, at a temperature of 240-600C, a pressure of 0.05-10 MPa, at a space velocity of 0.5-200 h$^{-1}$ and a hydrogen/hydrocarbons molar ratio of 0.5-12, in the presence of a catalyst containing a matrix, a mordenite, cerium and at least one metal from group VIII.

14. A process according to claim 13, operating at a temperature of 350-510C, a pressure of 0.2 to 3 MPa, at a space velocity of 2-100 h$^{-1}$ and a hydrogen/hydrocarbons molar ratio of 2 to 6.

15. A process according to claim 13 or claim 14, in which the catalyst contains 2-98% by weight of mordenite, 0.1-40% by weight of cerium, and 0.01-3% by weight of at least one metal from group VIII, the percentages being with respect to the total catalyst weight.

16. A process according to claim 15, in which the catalyst contains 7-40% by weight of cerium.

17. A process according to any one of claims 13 to 16, in which the cerium is deposited on the mordenite.

18. A process according to any one of claims 13 to 17, in which the mordenite is a large pore mordenite with an Si/Al

atomic ratio in the range 4.5 to 100.

19. A process according to any one of claims 13 to 18, in which the matrix is selected from the group constituted by alumina, magnesia, silica-alumina and clays.

20. A process according to any one of claims 13 to 19, in which the noble metal is selected from the group constituted by palladium and platinum.

21. A process according to any one of claims 13 to 20, in which cerium is deposited the mordenite by dry impregnating mordenite with an organic, aqueous or alcoholic solution of at least one cerium compound.